# EUROPÄISCHE PATENTSCHRIFT

(11) **EP 1 476 130 B1**
(45) Veröffentlichungstag und Bekanntmachung des Hinweises auf die Patenterteilung: **30.09.2009**
(21) Anmeldenummer: 03704565.5
(22) Anmeldetag: 07.02.2003
(51) Int. Cl.: A61K 8/34, A61Q 19/00

(54) **VERWENDUNG VON BESTIMMTEN DIOLEN ZUR REDUZIERUNG DER VISKOSITÄT VON KOSMETISCHEN UND/ODER DERMATOLOGISCHEN ZUBEREITUNGENM**
USE OF SPECIFIC DIOLS FOR REDUCING THE VISCOSITY OF COSMETIC AND/OR DERMATOLOGIC COMPOSITIONS
UTILISATION DE DIOLS SPECIFIQUES POUR RÉDUIRE LA VISCOSITÉ DES COMPOSITIONS COSMÉTIQUES ET/OU DERMATOLOGIQUES

(30) Priorität: 08.02.2002 DE 10205190
(43) Veröffentlichungstag der Anmeldung: 17.11.2004
(73) Patentinhaber: Beiersdorf AG, 20245 Hamburg (DE)
(72) Erfinder: KRÖPKE, Rainer, 22869 Schenefeld (DE); NIELSEN, Jens, 24558 Henstedt-Ulzburg (DE); WOLTER, Kathrin, 22251 Hamburg (DE)
(86) Internationale Anmeldenummer: PCT/EP2003/001204
(87) Internationale Veröffentlichungsnummer: WO 2003/066012

(56) Entgegenhaltungen:
- EP-A- 0 922 448
- EP-A- 1 283 031
- WO-A-00/56277
- WO-A-97/14398
- WO-A-99/56715
- FR-A- 2 780 283
- US-A- 5 798 111
- US-A- 5 827 520
- US-A- 6 113 888
- US-A1- 2001 051 169

## Beschreibung

Die vorliegende Erfindung betrifft kosmetische und/oder dermatologische Zubereitungen enthaltend ein oder mehrere Polyole in einer Konzentration von 0,1 bis 20 Gewichts-%, ein oder mehrere Diole aus der Gruppe 2-Methyl-1,3-propandiol, Pentandiol, Hexandiol in einer Konzentration von 0,1 bis 25 Gewichts-%, jeweils bezogen auf das Gesamtgewicht der Zubereitung, und deren Verwendung.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Aufgabe der kosmetischen Hautpflege ist es, die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) zu stärken oder wiederherzustellen.

Ziel der Hautpflege ist es ferner, den durch das tägliche Waschen verursachten Fettverlust der Haut auszugleichen. Dies ist gerade dann wichtig, wenn das natürliche Regenerationsvermögen nicht ausreicht.

Eine zentrale Aufgabe der Hautpflege besteht in der Hautbefeuchtung. Der Feuchtigkeitsgehalt der Haut hat einen bedeutetenden Einfluss auf ihr Aussehen und ihren Gesundheitszustand: Je höher der Wasserverlust, desto rauher und spröder wird die Haut. Ihre Elastizität und Plastizität läßt nach, die Unversehrtheit ist nicht mehr gewährleistet. Um die Haut zu befeuchten werden kosmetischen und dermatologischen Zubereitungen Feuchthaltesubstanzen (engl. Moisturizer) zugesetzt, die das Wasserbindevermögen der Homschicht unterstützen. Zu den klassischen Hautbefeuchtungsmitteln, die in fast allen kosmetischen und dermatologischen Zubereitungen eingesetzt werden, zählen die Polyole wie Glycerin und Sorbit. Daneben kommen auch andere Verbindungen zum Einsatz wie ethoxylierte Polyole und hydrolysierte Proteine. Außerdem finden Komponenten des natürlichen Feuchthaltefaktors der Haut (engl. Natural Moisturizing Factor, NMF) z.B. Harnstoff und bestimmte Aminosäuren Anwendung.

Ein großer Nachteil am Stand der Technik besteht in den negativen sensorischen Eigenschaften kosmetischer und/oder dermatologischer Zubereitungen die polyolhaltige Feuchthaltemittel enthalten. Diese fühlen sich in der Regel auf der Haut klebrig und schmierig an und machen die entsprechenden Produkte für den Verbraucher unattraktiv. Um dies negativen Eigenschaften zu unterdrücken, müssen den Zubereitungen teure Silikonöle und Lipide zugesetzt werden, welche die Herstellungskosten der Kosmetika/Dermatika erhöhen.

Es war daher die Aufgabe der vorliegenden Erfindung diesen Nachteil am Stande der Technik zu beseitigen und kostengünstige kosmetische und/oder dermatologische Zubereitungen zu entwickeln, deren sensorische Eigenschaften deutlich verbessert sind. Insbesondere sollten sich die Zubereitungen nicht klebrig anfühlen. Der Anteil an Silikonölen und anderen die Klebrigkeit reduzierenden Lipiden sollte verringert werden.

Kosmetische und dermatologische Zubereitungen werden weltweit in den unterschiedlichsten Klimazonen eingesetzt und von den Verbrauchern konsumiert. Da die Entwicklung von Kosmetika bzw. Dermatika recht aufwendig und teuer ist, versucht man zunehmend Rezepturen zu entwickeln, die global unter den unterschiedlichsten Klimabedingungen eingesetzt werden können. Die Temperaturstabilität dieser hoch komplexen, metastabilen Gemische ist jedoch relativ begrenzt. Bei tiefen Temperaturen von unter 5 °C nimmt ihre Viskosität so stark zu, dass die Zubereitungen kaum noch angewendet oder verarbeitet werden können. Auch neigen die Produkte bei tiefen Temperaturen und größeren Temperaturschwankungen, wie sie beispielsweise beim Transport auftreten können, zur Wasserabscheidung und Phasentrennung.

Es war daher eine weitere Aufgabe der vorliegenden Erfindung temperaturstabile kosmetische und dermatologische Zubereitungen zu entwickeln, die aufgrund ihrer geringen Viskosität auch bei tiefen Temperaturen noch anwendbar und zu verarbeiten sind.

Überraschend werden diese Aufgaben gelöst durch kosmetische und/oder dermatologische Zubereitungen enthaltend
a) ein oder mehrere Polyole in einer Konzentration von 0,1 bis 20 Gewichts-%,
b) ein oder mehrere Diole aus der Gruppe 2-Methyl-1,3-propandiol, Pentandiol,
Hexandiol in einer Konzentration von 0,1 bis 25 Gewichts-%,
jeweils bezogen auf das Gesamtgewicht der Zubereitung.

Die Zubereitungen zeichnen sich durch eine hohe Temperaturstabilität und geringer Viskosität bei tiefen Temperaturen aus. Außerdem haben sie ausgezeichnete sensorische Eigenschaften wie ein geringes Klebrigkeitsgefühl auf der Haut.

Erfindungsgemäß ist die Verwendung von Diolen aus der Gruppe 2-Methyl- 1,3 propandiol, Pentandiol, Hexandiol zur Reduzierung der Viskosität von Kos- metischen und/oder dermatologischen zubereitungen bei Temperaturen unter 10°C

Es ist erfindungsgemäß vorteilhaft die Polyole in einer Konzentration von 0,1 bis 20 Gewichts-% und insbesondere von 1 bis 15 Gewichts-% bezogen auf das Gesamtgewicht der Zubereitung einzusetzen.

Die erfindungsgemäß besonders bevorzugten Polyole sind Glycerin, Propylenglykol und Butylenglykol.

Außerdem ist es erfindungsgemäß vorteilhaft die Diole in einer Konzentration von 1 bis 15 Gewichts-% und insbesondere von 3 bis 8 Gewichts-% bezogen auf das Gesamtgewicht der Zubereitung einzusetzen.

Dabei ist erfindungsgemäß von Vorteil, wenn das Verhältnis von Polyolen zu Diolen in den erfindungsgemäßen kosmetischen und/oder dermatologischen Zubereitungen, von 5 : 1 bis 1 : 5, insbesondere von 3 : 1 bis 1 : 3 und ganz besonders bevorzugt von 2 : 1 bis 1 : 1 beträgt.

Als erfindungsgemäß besonders bevorzugtes Diol wird dabei 2-Methyl-1,3-propandiol eingesetzt. 2-Methyl-1,3-propandiol [INCI: Methyl Propanediol, z.B. MPDiol (Lyondell)] ist ein geruchloses Feuchthaltemittel mit der folgenden Struktur:

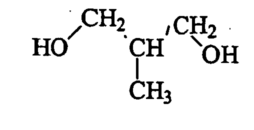

Die Zubereitungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Formulierungen können vorzugsweise auch eine Mikroemulsion, eine Feststoff-Emulsionen (d. h. eine Emulsion, welche durch Feststoffe stabilisiert ist, z. B. eine Pickering-Emulsion), eine sprühbare Emulsion oder eine Hydrodispersion sein. Vorteilhafte Ausführungsformen der erfindungsgemäßen Zubereitungen stellen beispielsweise Salben, Cremes und Lotionen dar. Des Weiteren können die Formulierungen im Sinne der vorliegenden Erfindung auch wasserfreie Systeme (Puder, Öle oder Wachsstifte) oder ölfreie Systeme (wässrige, wässrig/alkoholisch oder nur alkoholische Lösungen) sein. Hierzu können die jeweils entsprechenden Emulgatoren eingesetzt werden.

Die kosmetische und/oder dermatologische Zubereitungen können kosmetische Hilfsstoffe enthalten, wie sie üblicherweise in solchen Zubereitungen verwendet werden, z. B. Emulgatoren, Konservierungsmittel, Konservierungshelfer, Bakterizide, UV-Lichtschutzfilter, Parfüme, die Haut bleichende Mittel, Selbstbräuner, Repellentien, Substanzen zum Verhindern des Schäumens, Farbstoffe, Pigmente, die eine färbende Wirkung haben oder als UV-Lichtschutzfilter wirken, Verdickungsmittel, weitere anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polymere, Schaumstabilisatoren, Elektrolyte, organische Lösungsmittel oder Silikonderivate.

Die Wasserphase der Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, weitere Diole oder Polyole niedriger C-Zahl sowie deren Ether, vorzugsweise Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Dihydoxyaceton sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Ferner vorteilhaft sind Copolymere aus C₁₀₋₃₀-Alkylacrylaten und einem oder mehreren Monomeren der Acrylsäure, der Methacrylsäure oder deren Ester.

Vorteilhaft sind Verbindungen, die die INCI-Bezeichnung "Acrylates/C₁₀₋₃₀ Alkyl Acrylate Crosspolymer" tragen. Insbesondere vorteilhaft sind die unter den Handelsbezeichnungen Pemulen TR1 und Pemulen TR2 bei der B. F. Goodrich Company erhältlichen.

Vorteilhaft sind Verbindungen, die die INCI-Bezeichnung AmmoniumacryloyldimethyltaurateNinylpyrrolidoncopolymere tragen.

Erfindungsgemäß vorteilhaft weisen das oder die AmmoniumacryloyldimethyltaurateNinylpyrrolidoncopolymere die Summenformel [C₇H₁₆N₂SO₄]ₙ [C₆H₉NO]ₘ, auf, einer statistischen Struktur wie folgt entsprechend

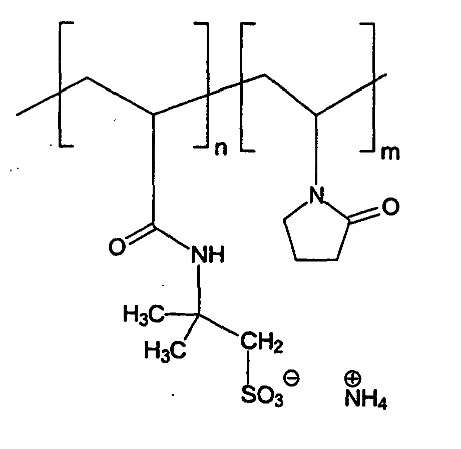

Bevorzugte Spezies im Sinne der vorliegenden Erfindung sind in den Chemical Abstracts unter den Registratumummern 58374-69-9, 13162-05-5 und 88-12-0 abgelegt und erhältlich unter der Handelsbezeichnung Aristoflex® AVC der Gesellschaft Clariant GmbH.

Vorteilhaft sind ferner Copolymere/Crosspolymere umfassend Acryloyldimethyl Taurate, wie beispielsweise Simugel ® EG oder Simugel ® EG von der Gesellschaft Seppic S.A.

Weitere erfindungsgemäß vorteilhaft zu verwendende Verdickungsmittel sind auch in Wasser lösliche oder dispergierbare anionische Polyurethane. Vorteilhaft im Sinne der vorliegenden Erfindung sind z. B. Polyurethan-1 und/oder Polyurethan-4.

Besonders vorteilhafte Polyurethane im Sinne der vorliegenden Erfindung sind die unter der Handelsbezeichnung Avalure^{™} UR bei der B. F. Goodrich Company erhältlichen Typen, wie beispielsweise Avalure^{™} UR 445, Avalure^{™} UR 450 und dergleichen. Ferner vorteilhaft im Sinne der vorliegenden Erfindung ist auch das unter der Handelsbezeichnung Luviset Pur bei der BASF erhältliche Polyurethan.

Die kosmetische und/oder dermatologische Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9).

Die Ölphase der erfindungsgemäßen kosmetischen und/oder dermatologischen Zubereitungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, Cocoglyceride (z. B. Myritol® 331 von Henkel), C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hattbilte BHB* bei der Fa. CP Hall erhältliche), Hexadecylbenzoat und Butyloctylbenzoat und Gemische davon (*Hallstar AB*) und/oder Diethylhexylnaphthalat (*Haftbrite TQ von CP Hall oder Corapan*®*TQvon Haarmann & Reimer*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 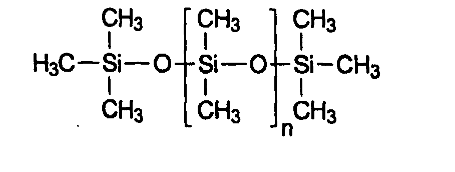 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), lodopropylbutyl-carbamate (z. B. die unter den Handelsbezeichnungen Glydant-2000, Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Ethylhexyloxyglycerin, Glycine Soja etc.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen und/oder dermatologischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin.

Erfindungsgemäße Rezepturen, welche z. B. bekannte Antifaltenwirkstoffe wie Flavonglycoside (insbesondere *a*-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Kosmetische und/oder dermatologische Zubereitungen im Sinne der vorliegenden Erfindung können vorteilhaft mindestens eine UV-A-, UV-B- und/oder Breitbandfiltersubstanz enthalten. Die Zubereitungen können, obgleich nicht notwendig, gegebenenfalls auch weitere organische und/oder anorganische Pigmente als UV-Filtersubstanzen enthalten, welche in der Wasser- und/oder der Ölphase vorliegen können.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicytat, Octylsalicylat, INCI: Octyl Salicylate) und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxy-cinnamat, INCI: Isoamyl p-Methoxycinnamate).

Vorteilhafte UV-A-Filtersubstanzen im Sinne der vorliegenden Erfindung sind Dibenzoyl-methanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-Di(2-oxo-10-sulfo-3-bornylidenmethyl)-benzol (auch: 3,3'-(1,4-Phenylendimethylene)-bis-(7,7-dimethyl-2-oxo-bicyclo-[2.2.1]hept-1-ylmethansulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bomylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bomylidenmethyl)benzolsulfonsäure, 2-Methyl-5-(2-oxo-3-bomylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist;
- Diethylhexylbutylamidotriazon (INCI: Diethylhexylbutamidotriazone), welches unter der Handelsbezeichnung UVASORB HEB bei Sigma 3V erhältlich ist;
4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird.

Die weiteren UV-Filtersubstanzen können öllöslich oder wasserlöslich sein.

Vorteilhafte öllösliche UV-B- und/oder Breitband-Filtersubstanzen im Sinne der vorliegenden Erfindung sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.
■ (3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Weitere erfindungsgemäß vorteilhaft zu verwendende Lichtschutzfiltersubstanzen sind das Ethylhexyl-2-cyano-3,3-diphenylacrylat (Octocrylen), welches von BASF unter der Bezeichnung Uvinul^{®} N 539 erhältlich ist und Hydroxybenzophenone wie der 2-(4'-(Diethylamino)-2'-hydoxybenzoyl)-benzoesäurehexylester.

Besonders vorteilhafte Zubereitungen im Sinne der vorliegenden Erfindung, die sich durch einen hohen bzw. sehr hohen UV-A- und/oder UV-B-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan], Phenylen-1,4-bis-(2-benzimidazyl)-3,3'-5,5'-tetrasulfonsäure und/oder ihre Salze, das 1,4-di(2-oxo-10-Sulfo-3-bomylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Bevorzugte weitere anorganische Pigmente sind Metalloxide und/oder andere in Wasser schwerlöslich oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Ferner kann es gegebenenfalls von Vorteil sein, Filmbildner in die erfindungsgemäßen Zubereitungen einzuarbeiten, beispielsweise um die Wasserfestigkeit der Zubereitungen zu verbessern oder die UV-Schutzleistung zu erhöhen (UV-A- und/oder UV-B-Boosting). Geeignet sind sowohl wasserlösliche bzw. dispergierbare als auch fettlösliche Filmbildner, jeweils einzeln oder in Kombination miteinander.

Vorteilhafte wasserlöslich bzw. dispergierbare Filmbildner sind z. B. Polyurethane (z. B. die Avalure® -Typen von Goodrich), Dimethicone Copolyol Polyacrylate (Silsoft Surface® von der Witco Organo Silicones Group), PVPNA (VA = Vinylacetat) Copolymer (Luviscol VA 64 Powder der BASF) etc.

Vorteilhafte fettlösliche Filmbildner sind z. B., die Filmbildner aus der Gruppe der Polymere auf Basis von Polyvinylpyrrolidon (PVP)

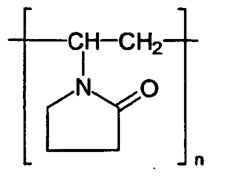

Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Make-up-Entferner, Tages- oder Nachtcreme usw. Es ist gegebenenfalls möglich und vorteilhaft, die erfindungsgemäßen Zusammensetzungen als Grundlage für pharmazeutische Formulierungen zu verwenden. Generell ist die Verwendung von erfindungsgemäßen Zubereitungen als Salbe, Creme, Milch, Lotion oder Spray zum Schutze der Haut und/oder der Hautanhanggebilde und/oder vor UV-Strahlung erfindungsgemäß.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **W/O-Emulsionen** | | | | | |
|---|---|---|---|---|---|
| | **1** | **2** | **3** | **4** | **5** |
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 7,5 |
| Vaseline | 1,0 | --- | 1,5 | --- | --- |
| hydrierte Kokosglyceride | 0,5 | 0,1 | 1,5 | --- | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,04 | --- | --- | 0,2 | 0,05 |
| Dicaprylylcarbonat | 0,1 | 0,05 | 0,15 | 0,5 | 1,0 |
| Magnesiumsulfat | 0,5 | 0,6 | 0,5 | 0,7 | 1,0 |
| Glycerin | --- | 5,0 | --- | 15,0 | 5 |
| Propylenglykol | 5,0 | --- | 10,0 | --- | --- |
| Butylenglykol | --- | --- | --- | --- | 5,0 |
| 2-Methyl-1,3-propandiol | 5,0 | 2,5 | 10,0 | 8,5 | 1,0 |
| Zitronensäure | 0,2 | 0,1 | 0,2 | 0,3 | 1,0 |
| Parfum | q,s, | q,s, | q,s, | q,s, | q,s, |
| Ethanol | 2,0 | --- | 5,0 | --- | 10,0 |
| Capryl-/Caprinsäuretriglycerid | 2,0 | 2,5 | 3,0 | 5,0 | 0,5 |
| Methylparaben | 0,4 | 0,15 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| Wasser | ad | ad | ad | ad | ad |
| | 100 | 100 | 100 | 100 | 100 |

| **W/O- Emulsionen** | | | | | |
|---|---|---|---|---|---|
| | **6** | **7** | **8** | **9** | **10** |
| PEG-30 Dipolyhydroxystearat | --- | 0,5 | 0,25 | --- | 3,0 |
| Lanolin Alcohol | 1,0 | 1,5 | 1,75 | 3,0 | -- |
| Paraffinöl | 12,5 | 10,0 | 8,0 | 5,0 | 17,5 |
| Vaseline | 8,0 | 6,0 | 5,0 | 2,0 | 2,5 |
| 2-Methyl-1,3-propandiol | 5,0 | 2,5 | 15,0 | 7,5 | 10,0 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Magnesiumsulfat | 0,5 | 0,6 | 0,5 | 0,7 | 1,0 |
| Glycerin | 3,0 | 5,0 | --- | --- | 2,0 |
| Propylenglykol | 5,0 | --- | 10,0 | --- | --- |
| Butylenglykol | --- | --- | --- | 3,0 | 5,0 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Methylparaben | 0,4 | 0,15 | 0,05 | 0,3 | 0,4 |
| Propylparaben | 0,3 | 0,4 | 0,25 | 0,15 | --- |
| Wasser | ad | ad | ad | ad | ad |
| | 100 | 100 | 100 | 100 | 100 |

| **W/O-Emulsionen** | | | | | |
|---|---|---|---|---|---|
| | **11** | **12** | **13** | **14** | **15** |
| Cetyldimethiconecopolyol | 1,0 | --- | --- | 3,0 | 5,0 |
| Cylomethicon + PEG/PPG-18/18 Dimethicon | 10,0 | 12,5 | 25 | --- | --- |
| Cyclomethicon | 12,5 | 15 | 8 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | --- | 2,0 | 3,0 | 15 |
| Propylenglykol | 5,0 | 5,0 | 5,0 | --- | --- |
| Butylenglykol | --- | --- | 4,0 | --- | 2,0 |
| 2-Methyl-1,3-propandiol | 15,0 | 2,5 | 10,0 | 8,5 | 1,0 |
| Zitronensäure | 0,2 | 0,1 | 0,2 | 0,3 | 1,0 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Cetyldimethicon | 0,5 | --- | 0,7 | --- | --- |
| lodopropynylbutylcarbamat | --- | --- | 0,05 | --- | 0,1 |
| modifizierte Stärke | --- | 2,5 | --- | 0,15 | --- |
| Wasser | ad | ad | ad | ad | ad |
| | 100 | 100 | 100 | 100 | 100 |

| **W/S- Emulsionen** | | | | | |
|---|---|---|---|---|---|
| | **16** | **17** | **18** | **19** | **20** |
| Cetyldimethiconecopolyol | 1,0 | --- | --- | 3,0 | 5,0 |
| Cylomethicon + PEG/PPG-18/18 Dimethicon | 10,0 | 12,5 | 25 | --- | --- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| 2-Methyl-1,3-propandiol | 5,0 | 2,5 | 10,0 | 8,5 | 1,0 |
| Glycerin | 3,0 | --- | 2,0 | 3,0 | 15 |
| Propylenglykol | 5,0 | 5,0 | 5,0 | --- | --- |
| Butylenglykol | --- | --- | 4,0 | --- | 2,0 |
| Natriumchlorid | 2,0 | 0,6 | 2,5 | 0,7 | 1,0 |
| Sorbitol | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Stearyldimethicon | 0,5 | --- | 0,7 | --- | --- |
| lodopropynylbutylcarbamat | --- | --- | 0,05 | --- | 0,1 |
| modifizierte Stärke | --- | 2,5 | --- | 0,15 | --- |
| Wasser | ad | ad | ad | ad | ad |
| | 100 | 100 | 100 | 100 | 100 |

| **Öl-in-Wasser- Emulsionen** | | | | | |
|---|---|---|---|---|---|
| | **21** | **22** | **23** | **24** | **25** |
| Glycerylsterat | 1,0 | --- | --- | 3,0 | 5,0 |
| PEG-40-Stearat | 10,0 | --- | 5 | --- | --- |
| Triglycerinmethylglucosedistearat | --- | 5,5 | --- | --- | 2,5 |
| Sorbitanstearat | --- | 1,5 | 3 | --- | --- |
| Cyclomethicon | 1 | 2,5 | 5 | 7,5 | 3 |
| Dimethicon | 5,0 | 13,0 | 5,0 | 12,0 | 15,0 |
| Behenylalkohol | 0,1 | --- | 0,2 | 0,5 | --- |
| Stearylalkohol | --- | 1 | --- | 1 | --- |
| Cetylstearylalkohol | --- | --- | 0,1 | 1 | --- |
| hydriertes Polyisobuten | 0,5 | 0,75 | 1,0 | 2,0 | 0,25 |
| Octyldodecanol | 0,5 | 1,0 | 0,75 | 3,0 | 0,25 |
| 2-Methyl-1,3-propandiol | 5,0 | 2,5 | 10,0 | 8,5 | 1,0 |
| Panthenol | 0,5 | 1,0 | 0,75 | 0,25 | 0,1 |
| Glycerin | 3,0 | --- | 2,0 | 3,0 | 15 |
| Propylenglykol | 5,0 | 5,0 | 5,0 | --- | --- |
| Butylenglykol | --- | --- | 4,0 | --- | 2,0 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| modifizierte Stärke | --- | 2,5 | --- | 0,15 | --- |
| Wasser | ad 100 | ad 100 | ad 100 | ad 100 | ad 100 |

| **Öl-in-Wasser- Emulsionen** | | | | | |
|---|---|---|---|---|---|
| | **26** | **27** | **28** | **29** | **30** |
| Polyethylenglycol(21)stearylether | 1 | --- | 2,5 | 2 | 1,5 |
| Polyethylenglycol(2)stearylether | 1 | --- | 1,5 | 3 | 1,5 |
| Cetearylglucosid | --- | 8 | --- | --- | --- |
| Cyclomethicon | 2,5 | 3 | 12,5 | 2 | 8,0 |
| Dimethicon | 5,0 | 13,0 | 5,0 | 12,0 | 5,0 |
| Behenylalkohol | 0,3 | 0,5 | --- | 0,1 | --- |
| Stearylalkohol | 0,3 | --- | --- | 0,2 | --- |
| Cetylstearylalkohol | 0,3 | 0,5 | --- | --- | 0,25 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| 2-Methyl-1,3-propandiol | 15,0 | 2,5 | 10,0 | 8,5 | 25,0 |
| Panthenol | 0,5 | 1,0 | 0,75 | 0,25 | 0,1 |
| Glycerin | 3,0 | --- | 2,0 | 3,0 | 15 |
| Propylenglykol | 5,0 | 5,0 | 5,0 | --- | --- |
| Butylenglykol | --- | --- | 4,0 | --- | 2,0 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | |
| Iodopropynylbutylcarbamat | --- | --- | 0,05 | --- | 0,1 |
| modifizierte Stärke | --- | 2,5 | --- | 0,15 | --- |
| Wasser | ad 100 | ad 100 | ad 100 | ad 100 | ad 100 |

## Patentansprüche

1. Verwendung von einem oder mehreren Diolen aus der Gruppe 2-Methyl-1,3-propandiol, Pentandiol, Hexandiol zur Reduzierung der Viskosität von kosmetischen und/oder dermatologischen Zubereitungen bei Temperaturen unter 10 °C.

2. Verwendung nach Anspruch 1 in einer kosmetischen und/oder dermatologischen Zubereitung enthaltend
a) ein oder mehrere Polyole in einer Konzentration von 0,1 bis 20 Gewichts-%,
b) ein oder mehrere Diole aus der Gruppe 2-Methyl-1,3-propandiol, Pentandiol, Hexandiol in einer Konzentration von 0,1 bis 25 Gewichts-%,
jeweils bezogen auf das Gesamtgewicht der Zubereitung.

3. Verwendung nach Anspruch 2, **dadurch gekennzeichnet, dass** das Verhältnis von Polyolen zu Diolen von 1 : 10 bis 10 : 1 beträgt.

4. Verwendung nach einem der Ansprüche 2 oder 3, **dadurch gekennzeichnet, dass** als Diol 2-Methyl-1,3-propandiol eingesetzt wird.

5. Verwendung nach einem der Ansprüche 2 bis 4, **dadurch gekennzeichnet, dass** als Polyol Glycerin, Propylenglykol oder
Butylenglykol eingesetzt wird.

## Claims

1. Use of one or more diols from the group consisting of 2-methyl-1,3-propanediol, pentanediol, hexanediol for reducing the viscosity of cosmetic and/or dermatological formulations at a temperature below 10 °C.

2. Use according to Claim 1 in a cosmetic and/or dermatological formulation comprising
a) one or more polyols in a concentration of 0.1 to 20 % by weight,
b) one or more diols from the group consisting of 2-methyl-1,3-propanediol, pentanediol, hexanediol in a concentration of 0.1 to 25 % by weight,
in each case based on the total weight of the formulation.

3. Use according to Claim 2, **characterized in that** the ratio of polyols to diols is from 1 : 10 to 10 : 1.

4. Use according to one of Claims 2 or 3, **characterized in that** 2-methyl-1,3-propanediol is employed as the diol.

5. Use according to one of Claims 2 to 4, **characterized in that** glycerol, propylene glycol or butylene glycol is employed as the polyol.

## Revendications

1. Utilisation d'un ou plusieurs diols choisis dans le groupe constitué par le 2-méthyl-1,3-propanediol, le pentanediol, l'hexanediol, pour la réduction de la viscosité de préparations cosmétiques et/ou dermatologiques à des températures inférieures à 10 °C.

2. Utilisation selon la revendication 1, dans une préparation cosmétique et/ou dermatologique contenant
a) un ou plusieurs polyols à une concentration de 0,1 à 20 % en poids,
b) un ou plusieurs diols choisis dans le groupe constitué par le 2-méthyl-1,3-propanediol, le pentanediol, l'hexanediol, à une concentration de 0,1 à 25 % en poids,
chaque fois par rapport au poids total de la préparation.

3. Utilisation selon la revendication 2, **caractérisée en ce que** le rapport des polyols aux diols va de 1:10 à 10:1.

4. Utilisation selon la revendication 2 ou 3, **caractérisée en ce qu'**on utilise comme diol le 2-méthyl-1,3-propanediol.

5. Utilisation selon l'une quelconque des revendication 2 à 4, **caractérisée en ce qu'**on utilise comme polyol le glycérol, le propylèneglycol ou le butylèneglycol.
